# EUROPÄISCHE PATENTSCHRIFT

(11) **EP 0 994 689 B1**
(45) Veröffentlichungstag und Bekanntmachung des Hinweises auf die Patenterteilung: **25.09.2002**
(21) Anmeldenummer: 98929442.6
(22) Anmeldetag: 10.06.1998
(51) Int. Cl.: A61F 13/58

(54) **HYGIENEARTIKEL ZUM EINMALIGEN GEBRAUCH**
DISPOSABLE HYGIENIC ARTICLE
ARTICLE HYGIENIQUE JETABLE

(30) Priorität: 01.07.1997 DE 19727916
(43) Veröffentlichungstag der Anmeldung: 26.04.2000
(73) Patentinhaber: Paul Hartmann Aktiengesellschaft, 89522 Heidenheim (DE)
(72) Erfinder: MALOWANIEC, Krzysztof, D., D-89522 Heidenheim (DE)
(74) Vertreter: Friz, Oliver
(86) Internationale Anmeldenummer: EP9803512
(87) Internationale Veröffentlichungsnummer: WO99001100

(56) Entgegenhaltungen:
- EP-A- 0 247 855
- EP-A- 0 324 577
- EP-A- 0 719 532
- WO-A-96/25905
- DE-U- 29 711 430

## Beschreibung

Die Erfindung betrifft einen Hygieneartikel zum einmaligen Gebrauch, insbesondere eine Windel, ein Inkontinenzprodukt oder eine Inkontinenzeinlage, mit einem Vorderbereich, einem Rückbereich und einem dazwischen liegenden im Schrittbereich des Benutzers des Hygieneartikels zu liegen kommenden Mittelbereich, die eine die körperabgewandte Außenseite bildende, zumindest abschnittsweise flüssigkeitsdichte Schicht und eine die körperzugewandte Innenseite bildende, zumindest im Bereich eines darunter angeordneten Saugkörpers flüssigkeitsdurchlässige Schicht aufweisen, und mit einem Befestigungssystem zum lösbaren Verbinden von Rückbereich und Vorderbereich des Hygieneartikels im an den Benutzer angelegten Zustand und zum Fixieren des Artikels im zusammengerollten oder gefalteten Zustand des gebrauchten Artikels beim Wegwerfen oder Entsorgen, wobei das Befestigungssystem einen ersten Befestigungsabschnitt mit Festhaltemitteln am Rückbereich und einen zweiten Befestigungsabschnitt mit Festhaltegegenmitteln am Vorderbereich aufweist, wobei der erste Befestigungsabschnitt beim Verbinden von Rückbereich und Vorderbereich am zweiten Befestigungsabschnitt positionierbar ist, damit die Festhaltemittel und Festhaltegegenmittel eine mechanische Halteverbindung eingehen, und wobei die Festhaltemittel des ersten Befestigungsabschnitts zusätzlich klebend ausgestaltet sind.

Zum Anlegen eines solchen Körperflüssigkeiten und - ausscheidungen aufnehmenden Hygieneartikels wird der Rückbereich und der Vorderbereich gegen den Körper des Benutzers angelegt, wobei Vorder- und Rückbereich hierbei mit ihren Seitenrändern wenigstens nahezu aneinander angrenzen oder einander überlappen, damit unter Verwendung des Befestigungssystems Vorder- und Rückbereich in einem möglichst optimalen Sitz des Artikels am Körper des Benutzers verbunden werden können, so dass ein Austreten von Körperflüssigkeiten oder -ausscheidungen unterstützt durch weitere Hilfsmittel, wie elastisch dehnbare Flüssigkeitssperren, -bündchen und geeignete Dimensionierung des Saugkörpers, verhindert wird. Zum Wechseln des benutzten Hygieneartikels gegen einen unbenutzten wird das Befestigungssystem gelöst, und der Körperflüssigkeiten und - ausscheidungen enthaltende Hygieneartikel wird vom Benutzer abgenommen. Um den benutzten Hygieneartikel zum Entsorgen handhabbarer zu machen, wird er üblicherweise vom Vorderbereich aus in Richtung auf den Rückbereich zusammengerollt oder - gefaltet, so dass ein greifbares Päckchen gebildet wird, das der Benutzer, die Eltern oder eine Pflegeperson manuell greifen und in einen Entsorgungsbehälter werfen kann. Um weiter zu verhindern, dass sich das gerollte oder gefaltete Päckchen beim Wegwerfen oder danach im Entsorgungsbehälter wieder entfaltet und zu einer Geruchsbelästigung sowie zum Austreten seines unangenehm riechenden Inhalts führt, wird das Befestigungssystem - wie eingangs bereits angedeutet - auch dazu benutzt, den Hygieneartikel in seinem zusammengerollten oder gefalteten Zustand zu fixieren. Hierbei wird in bekannter Weise der erste Befestigungsabschnitt derart auf die Außenseite des gerollten oder gefalteten Päckchens aufgedrückt, dass er dort anhaftet, klebt oder hakt und ein Aufrollen oder Entfalten des Hygieneartikels verhindert.

Der erste Befestigungsabschnitt am Rückbereich bekannter Hygieneartikel ist üblicherweise an einem sanduhrförmig nach den Seiten hin erweiterten Abschnitt des Rückbereichs, einer sog. Seitenklappe, oder an einer quer zur Längsrichtung, also zu den Seiten hin vorstehenden Befestigungslasche vorgesehen, die am Rückbereich bzw. an den Seitenklappen des Rückbereichs befestigt ist. Der zweite Befestigungsabschnitt am Vorderbereich ist entweder nicht genau definiert und von einer ansich beliebige mechanisch wirkende Festhaltegegenmittel aufweisenden Oberfläche gebildet, oder er erstreckt sich zumeist in Form eines bandförmigen quer verlaufenden Bereichs an einem Bundendabschnitt im Vorderbereich des Artikels. Der erste Befestigungsabschnitt kann also entsprechend der Körperform des Benutzers den Vorderbereich mehr oder weniger überlappend positioniert werden.

Zur Ausgestaltung des Befestigungssystems sind zahlreiche Lösungen bekannt geworden. Mit der EP 0 321 232 B1 wird beispielsweise vorgeschlagen, den ersten Befestigungsabschnitt in Form einer Lasche am Rückbereich so auszubilden, dass neben einem ersten die mechanisch wirkenden Festhaltemittel aufweisenden Teilabschnitt ein zweiter einen Klebstoffauftrag aufweisender Teilabschnitt vorgesehen ist. Hierbei dient der erste Teilabschnitt mit den mechanisch wirkenden Festhaltemitteln zum Schließen des Hygieneartikels im an den Benutzer angelegten Zustand, indem die mechanischen Festhaltemittel mit mechanisch wirkenden Festhaltegegenmitteln klettverschlussartig zusammenwirken. Der Klebstoffauftrag in dem zweiten Teilabschnitt dient dazu, die Lasche an der die körperabgewandte Außenseite bildenden Schicht des Artikels klebend zu fixieren. Wenn der benutzte Artikel vom Vorderbereich zum Rückbereich zusammengerollt oder gefaltet wird, so dass der zweite Befestigungsabschnitt innerhalb des Päckchens zu liegen kommt, so soll das Päckchen unter Zusammenwirken des klebenden zweiten Teilabschnitts mit der folienhaft glatten Außenoberfläche des Artikels fixiert werden. Dieses Befestigungssystem erweist sich aber insoweit als nachteilig, als der erste Befestigungsabschnitt, d.h. die Befestigungslaschen, entsprechend der zwei Teilabschnitte sehr groß bzw. lang ausgelegt sein müssen. Der Materialbedarf ist dementsprechend groß und die seitlich vorstehenden Laschen stören bei der Handhabung des Artikels und wirken ungefällig. Eine definierte Haltekraft des Befestigungssystems ist nicht erreichbar, da es beim Schließen zu einer mehr oder weniger großen Überlappung oder Berührung des klebend ausgebildeten zweiten Teilabschnitts mit der Umgebung des zweiten Befestigungsabschnitts an der Vorderseite und zu einer dementsprechend mehr oder weniger starken zusätzlichen klebenden Verbindung neben der bestimmungsgemäß mechanischen Verbindung der Festhaltemittel und Festhaltegegenmittel kommt. Diese Unbestimmtheit muss als nachteilig angesehen werden.

Mit der EP 0 321 234 A1 wird ein anderer Lösungsweg gegangen, indem an der die körperabgewandte Außenseite bildende Schicht ein Auftreffabschnitt mit mechanisch wirkenden Festhaltegegenmitteln separat vorgesehen ist, der in der zusammengerollten oder gefalteten Konfiguration des Artikels mit dem ersten Befestigungsabschnitt zusammenwirkt. Ein Klebstoffauftrag ist hier nicht vorgesehen. Die Anordnung dieses weiteren mechanisch wirkenden Auftreffabschnitts auf der Außenseite des Artikels ist aufwendig und die Handhabung beim Zusammenfalten des Artikels ist schwierig und erfordert Aufmerksamkeit, da die Befestigungsabschnitte exakt zueinander positioniert werden müssen. Gerade in dieser Situation kann diese Aufmerksamkeit aber erfahrungsgemäß kaum aufgebracht werden, da bei der Pflege von Kleinkindern häufig nur eine Hand zum raschen Zusammenfalten der Windel zur Verfügung steht und möglichst gleichzeitig ein unbenutzter Hygieneartikel dem Kleinkind untergeschoben werden sollte.

Aus der EP 0 393 953 B1 ist es bekannt, zum Befestigen eines Hygieneartikels in Form einer Monatsbinde an einem Kleidungsstück auch einen Klebstoffauftrag auf der dem Kleidungsstück zugewandten im Gebrauch unteren Außenseite der Binde zu verwenden. Damit die Binde an dem textilen Material des Kleidungsstücks unverrutschbar fixiert werden kann, sind auf der die Klebstoffschicht aufweisenden Außenseite der Binde sich verjüngende Vorsprünge vorgesehen, die in das textile Material eingedrückt werden und so die Binde unverschieblich festlegen. Eine derartige Ausbildung des ersten Befestigungsabschnitts des in Rede stehenden Hygieneartikels dürfte sich jedoch als nicht geeignet erweisen, da ein Zusammenwirken mit einer folienhaften körperabgewandten Außenseite des Artikels solchenfalls nicht möglich wäre.

Mit der WO 96/25905 wurde vorgeschlagen, den ersten Befestigungsabschnitt bzw. die mechanisch wirkenden hakenartigen Festhaltemittel und gleichzeitig auch den zweiten Befestigungsabschnitt mit mechanisch wirkenden Festhaltegegenmitteln teilweise mit einem Klebstoffauftrag zu versehen. Beim Verbinden von Rückteil und Vorderteil im an den Benutzer angelegten Zustand des Artikels sollen erklärtermaßen beide Verbindungsmechanismen, also mechanisch haltend oder hintergreifend einerseits und klebend andererseits in Kombination zusammenwirken, um die erwünschte Befestigung zu bilden. Dabei wird die zum Lösen der Verbindung erforderliche Kraft in eine auf die Ebene projizierte Scherkraft und eine senkrecht zur Ebene wirkende Abzugskraft zerlegt, welcherstere dem mechanischen Verbindungsmechanismus und welchletztere primär der Klebeverbindung zugeschrieben wird. Nach der Lehre dieser Druckschrift werden unter dem Begriff "Klebermaterial" solche Materialien verstanden, die eine klebende Verbindung nur mit anderen klebenden Materialien ausbilden. Dadurch, dass sowohl der erste als auch der zweite Befestigungsabschnitt zusätzlich mit einem solchen klebenden Material versehen ist, soll eine starke die mechanische Verbindung unterstützende Haftkraft erreicht werden. Das Problem des Fixierens des benutzten Hygieneartikels in zusammengerollter oder gefalteter Konfiguration wird in dieser Druckschrift nicht angesprochen.

Die WO-A-98/27922 veröffentlicht ein kombiniertes mechanisches und klebendes Verschlußsystem. Es wird jedoch kein Bezug auf die Peel-Off-Kraft dargelegt. Dieses Dokument ist nicht vorveröffentlicht (Art. 54(3) EPÜ).

Der vorliegenden Erfindung liegt die Aufgabe zugrunde, einen Hygieneartikel der eingangs beschriebenen Art dahingehend zu verbessern, dass beim Anlegen des Artikels an den Körper eines Benutzers stets ein korrekter Sitz des Artikels sowie eine sichere Verbindung von Vorder- und Rückbereich erreicht werden und dass der benutzte Artikel in zum Wegwerfen geeigneter Konfiguration in leicht handhabbarer Weise fixierbar ist.

Diese Aufgabe wird durch einen Hygieneartikel mit den eingangs genannten Merkmalen erfindungsgemäß dadurch gelöst,
a) dass die Festhaltegegenmittel (90) des zweiten Befestigungsabschnitts (64) klebstofffrei sind und zum Schließen des Hygieneartikels im an den Körper angelegten Zustand mit den Festhaltemitteln (72) des ersten Befestigungsabschnitts (62) eine mechanische Halteverbindung mit einer 1. Peel-Off-Kraft von mehr als 1 N/25mm ausbilden, und
b) dass die Festhaltemittel (72) des ersten Befestigungsabschnitts (62) derart klebend und die die Außenseite (42) bildende Schicht (44) zumindest in der Umgebung (100) des zweiten Befestigungsabschnitts (64) am Vorderbereich (4) derart glatt ausgebildet ist, dass die Festhaltemittel (72) des ersten Befestigungsabschnitts (62) dort mit einer 2. Peel-Off-Kraft haften, die geringer als die 1. Peel-Off-Kraft ist, und
c) dass die Festhaltemittel (72) des ersten Befestigungsabschnitts (62) derart klebend und die die Außenseite (42) bildende Schicht (44) zumindest im Mittelbereich (8) derart glatt ausgebildet ist, dass die Festhaltemittel (72) des ersten Befestigungsabschnitts (62) daran mit einer Peel-Off-Kraft von wenigstens 0.1 N/25mm klebend haften, um den Hygieneartikel in zusammengerollter oder -gefalteter Konfiguration zu fixieren.

Mit fortschreitender Entwicklung der Hygieneprodukte wird eine der Körperform immer besser entsprechende Gestaltung erreicht. Der Erfindung liegt u.a. die Erkenntnis zugrunde, dass gerade bei optimaler Gestaltung eine perfekte Passform des Hygieneprodukts und damit ein sicherer Auslaufschutz und ein angenehmes von Zwicken freies Traggefühl nur dann gegeben ist, wenn das Befestigungssystem, d.h. der erste und zweite Befestigungsabschnitt, in korrekter der Konfektionierung des Artikels entsprechender Weise positioniert und fixiert werden. Der Hygieneartikel wird also nach der Erfindung so ausgelegt, dass ein Benutzer des Hygieneartikels, die Eltern oder Pflegepersonal, beim Anlegen des Hygieneartikels gezwungen sind, das Befestigungssystem so zu benutzen, d.h. die Befestigungsabschnitte am Rück- und Vorderbereich so zueinander auszurichten und zu positionieren, dass die Befestigungsabschnitte einander wenigstens nahezu vollständig überdecken oder überlappen, um die erforderliche Haltekraft bereit zu stellen. Dies wird durch die vorstehend genannten erfindungsgemäßen Merkmale erreicht. Wird das Befestigungssystem nachlässig benutzt, so dass der erste Befestigungsabschnitt nur wenig mit dem zweiten Befestigungsabschnitt überlappt oder gar an dazu versetzter Stelle, beispielsweise unterhalb eines als bandförmiger Bund ausgebildeten zweiten Befestigungsabschnitts, zu liegen kommt, so wird durch die erfindungsgemäße Ausbildung des Befestigungssystems der Benutzer ohne Weiteres realisieren, dass bei dieser Positionierung keine ausreichende Befestigung des Systems erreicht wird. Er wird den ersten Befestigungsabschnitt von der soeben gewählten Stelle wieder lösen und an dem bei einiger Aufmerksamkeit ohne Weiteres identifizierbaren zweiten Befestigungsabschnitt positionieren.

Mit der Erfindung wurde desweiteren erkannt, dass bei der Verwendung des Befestigungssystems zum Fixieren des Hygieneartikels in einer zum Entsorgen geeigneten Konfiguration eine Peel-Off-Kraft von wenigstens 0,1 N/25 mm, vorzugsweise von 0,5 bis 1 N/25 mm, hinreichend ist. Ein weiterer Vorteil der erfindungsgemäßen Ausgestaltung ergibt sich daraus, dass zum Zwecke dieses Fixierens kein zusätzliches separates, schwer zu handhabendes Auftreffmittel vorgesehen werden muss, sondern dass der jeweilige erste Befestigungsabschnitt so ausgebildet ist, dass er an beliebiger Stelle an der Außenseite des Mittelbereichs, ggf. auch am Rückbereich, in der angegebenen Weise hinreichend haftet.

Es wird ausdrücklich darauf hingewiesen, dass die Beschaffenheit der die Außenseite bildenden Schicht über ihre ganze Fläche gleich sein kann. Solchenfalls hält der erste Befestigungsabschnitt in der Umgebung des zweiten Befestigungsabschnitts mit derselben 2. Peel-Off-Kraft von wenigstens 0,1N/25mm wie auf der übrigen Außenseite der Schicht, insbesondere des Mittelbereichs und des Rückbereichs.

Die vorstehend als Peel-Off-Kräfte bezeichneten Haltekräfte werden in der nachstehend beschriebenen Weise definiert und gemessen. Zum Test wird ein 25 mm breiter Teststreifen, eines Materials, welches den ersten Befestigungsabschnitt mit seinen Festhaltemitteln bildet, zur Verfügung gestellt. Dieser Teststreifen wird unter einem Anpressdruck von 2 kg unter Verwendung eines Überrollgeräts auf die zu testende Gegenfläche aufgebracht. Hierbei handelt es sich entweder um den zweiten Befestigungsabschnitt oder aber um die die Außenseite bildende Schicht des Produkts, welche die Umgebung des zweiten Befestigungsabschnitts und vorzugsweise auch die übrige Außenseite des Produkts bildet, wie z.Bsp. den Mittelbereich, auf den der erste Befestigungsabschnitt zum Entsorgen des zusammengerollten Hygieneartikels aufgebracht wird, um die zusammengerollte oder gefaltete Konfiguration zu fixieren. Diese Gegenfläche ist dabei auf einen starren Halter aufgebracht. Der Halter wird in einem Zugprüfgerät fixiert und der Teststreifen wird an einer Zugbacke geklemmt, so dass sich ein Abzugswinkel von 150° ergibt, der sich beim Abziehen geringfügig um einige wenige Grad verringert. Unter Messung der als Peel-Off-Kraft bezeichneten Haltekraft wird der Teststreifen, also der erste Befestigungsabschnitt, von der Gegenfläche mit konstanter Geschwindigkeit abgezogen. Die gemessene Peel-Off-Kraft wird als Funktion des Wegs aufgezeichnet.

Es ist nunmehr in ganz besonders vorteilhafter Weise möglich, die die Außenseite des Artikels bildende Schicht in ansich beliebiger Weise, insbesondere mit einer im wesentlichen glatten folienartigen Oberfläche oder mit einer faserigen, insbesondere textil anmutenden Oberfläche, auszubilden. Im ersteren Fall wird die noch verbleibende Haltekraft, welche im Bereich der Umgebung des zweiten Befestigungsabschnitts nicht zufriedenstellend, im Mittelbereich jedoch zu dem Zweck der Entsorgung noch hinreichend ist, im wesentlichen von der klebenden Haftwirkung bestimmt. Wenn die die Außenseite des Hygieneartikels bildende Schicht eine faserige, textil anmutende Oberfläche aufweist, so wird hierdurch der Einfluss der Klebewirkung reduziert und derjenige des mechanischen Verbindungsmechanismus in den Vordergrund gerückt. Solchenfalls wird die Außenseite des Artikels in der Umgebung des zweiten Befestigungsabschnitts am Vorderbereich gleichwohl noch so glatt ausgebildet sein, dass die dort auftretende 2. Peel-Off-Kraft vorzugsweise wesentlich unterhalb der 1. Peel-Off-Kraft liegt.

Es versteht sich, dass die mechanisch wirkenden Festhaltemittel des ersten Befestigungsabschnitts ganz oder teilweise mit Kleber überzogen sein können. Es wird desweiteren darauf hingewiesen, dass die mechanisch wirkenden Festhaltemittel auch aus klebendem Material bestehen können.

Bevorzugte Ausführungsformen der Festhaltemittel sowie der Ausbildung des zweiten Befestigungsabschnitts mit den Festhaltegegenmitteln und der die körperabgewandte Außenseite bildenden Schicht des Hygieneartikels ergeben sich aus den beigefügten Schutzansprüchen.

Als besonders vorteilhaft hat es sich erwiesen, wenn die Festhaltemittel von Vorsprüngen mit hintergreifenden oder hintergreifbaren Endbereichen gebildet sind und eine Geometrie bzw. Abmessung wie in den Ansprüchen angegeben aufweisen. Bei dem zweiten Befestigungsabschnitt handelt es sich um ein vorzugsweise entlang des Bundendabschnitts des Vorderbereichs verlaufendes flauschförmiges faseriges Bandmaterial. Es kann sich hierbei um ein textiles Material, wie beispielsweise ein Gewebe oder ein Gewirk handeln, wobei aber bevorzugtermaßen auf Vliesbasis bestehende Nonwoven-Materialien zum Einsatz kommen. Ein solches auf Vliesbasis hergestelltes flauschförmiges Material weist in der Regel eine zweischichtige Struktur auf mit einer Trägerschicht, die zum Beispiel aus einer Folie, einem Spinnvlies oder einem Meltblown-Vlies oder dergleichen bestehen kann, und einer die Festhaltegegenmittel bildenden Schlaufenschicht, zum Beispiel ein Kardenfließ oder ein Spinnvlies, in welche die Festhaltemittel des ersten Befestigungsabschnitts eingreifen und dort verhaken können.

Außerdem kann das flauschförmige Material ein Druckbild aufweisen, dass entweder auf die Trägerschicht oder direkt auf die Schlaufenschicht oder auf eine dritte, insbesondere folienartige Schicht aufgebracht werden kann, die dann bevorzugtermaßen zwischen der Trägerschicht und der Schlaufenschicht positioniert ist.

Es wurde festgestellt, dass für die Haltekraft zwischen dem ersten Befestigungsabschnitt des Rückbereichs und dem flauschförmigen Material des Vorderbereichs die Ausführung des flauschförmigen, faserigen Materials von besonderer Bedeutung ist. Faserstärke, Faserlänge, Flächengewicht des flauschförmigen faserigen Materials werden vorzugsweise nach den Angaben in den Schutzansprüchen ausgeführt.

Als besonders vorteilhaft hat es sich ergeben, wenn das flauschförmige faserige Material in einem regelmäßigen Muster angeordnete Schlaufen bildende Erhebungen mit den Abmessungen nach Angabe der beigefügten Schutzansprüche aufweist. Die Schlaufen bildenden Erhebungen können vorteilhafter Weise durch Heißsiegeln des flauschförmigen faserigen Materials gegen die Trägerschicht erreicht werden. Von der Firma 3M Deutschland GmbH, Neuss, DE kann ein Flachmaterialverbund aus einer folienartigen Trägerschicht und einer flauschförmigen Faserschicht bezogen werden, welche durch Anbringung von parallel zueinander erstreckten Heißsiegelprägungen ein paralleles endloses Wellenmuster von Erhebungen aufweist. Von der Firma Corovin GmbH, Peine, DE kann ein Flachmaterialverbund mit rautenförmigen Heißsiegelprägungen bezogen werden.

Bei der die körperabgewandte Außenseite bildenden Schicht, die eine glatte Oberfläche oder eine zumindest einen gewissen textilen, faserigen Charakter vermittelnde Oberfläche aufweisen kann, handelt es sich um eine Folie oder vorzugsweise um ein zumindest zweischichtiges, insbesondere dreischichtiges Vlies-Folien-Laminat. Das dreischichtige Laminat umfasst vorzugsweise eine die Außenseite bildende folienähnliche Meltblown-Schicht, eine sich daran anschließende Spinnvlies-Schicht und eine innenliegende Folienschicht (Spunbond-Meltblown-Laminat).

Weitere Merkmale, Einzelheiten und Vorteile der Erfindung ergeben sich aus den beigefügten Schutzansprüchen sowie der zeichnerischen Darstellung und nachfolgenden Beschreibung einer bevorzugten Ausführungsform des erfindungsgemäßen Hygieneprodukts. In der Zeichnung zeigt:
- Figur 1: eine Ansicht eines erfindungsgemäßen Hygieneartikels in Form einer Windel;
- Figur 2: eine Schnittansicht mit der Schnittebene II-II in Figur 1;
- Figur 3: eine perspektivische Ansicht einer Windel in an einen Benutzer angelegten Zustand;
- Figur 4: eine schematische Schnittansicht eines ersten Befestigungsabschnitts der Windel mit der Schnittebene IV-IV in Figur 1;
- Figur 5: eine vergrößerte Darstellung eines mechanisch wirkenden Festhaltemittels des ersten Befestigungsabschnitts nach Figur 4;
- Figur 6: eine vergrößerte Darstellung einer zweiten Ausführungsform eines mechanisch wirkenden Festhaltemittels
- Figur 7: eine Darstellung einer dritten Ausführungsform eines mechanisch wirkenden Festhaltemittels
- Figur 8: eine Schnittansicht eines zweiten Befestigungsabschnitts der Windel mit der Schnittebene VIII-VIII in Figur 1; und
- Figur 9: eine Schnittansicht durch eine die Außenseite der Windel bildende Schicht.

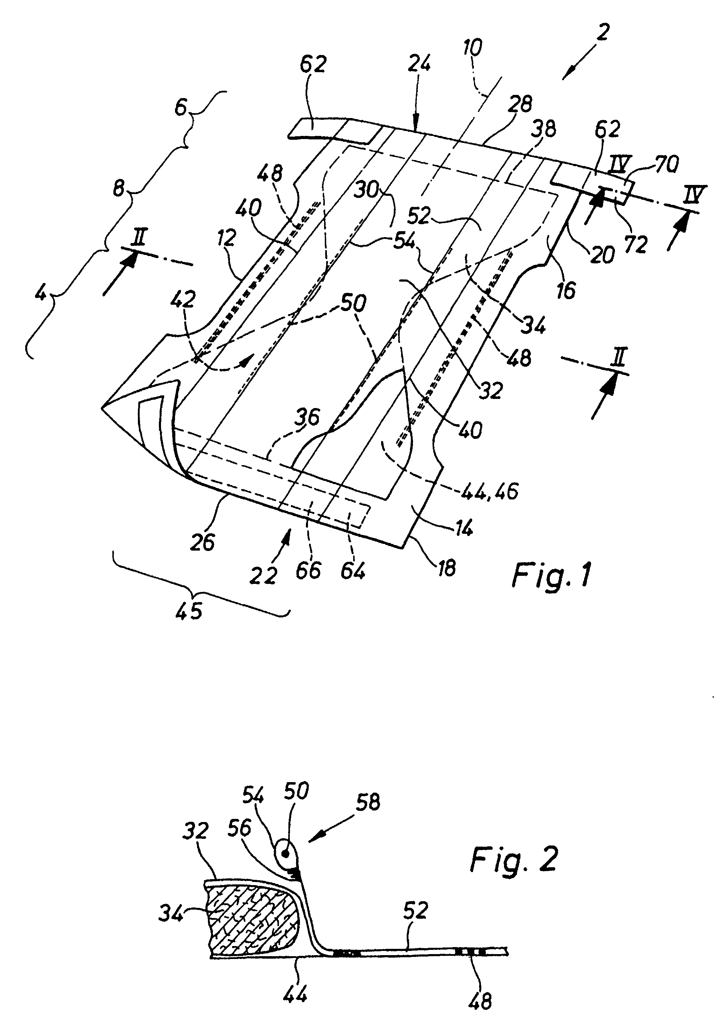

Figur 1 zeigt einen Hygieneartikel in Form einer Windel 2 für ein Kleinkind oder für inkontinente Personen. Die Windel 2 wird im Schrittbereich zwischen den Beinen eines Benutzers angeordnet und dann nach oben in Richtung auf die Hüften des Benutzers an den Körper angelegt und auf noch näher zu beschreibende Weise so fixiert, dass eine Art Höschen gebildet wird.

Die Windel besteht aus einem Vorderbereich 4, einem Rückbereich 6 und einem dazwischen liegenden, im Schrittbereich des Benutzers der Windel zu liegen kommenden Mittelbereich 8. Der Vorderbereich 4, der Mittelbereich 8 und der Rückbereich 6 sind in einer Längsrichtung 10 angeordnet. Der Mittelbereich weist zwei in Längsrichtung 10 verlaufende Längsränder 12 auf, die zum Vorderbereich 4 und zum Rückbereich 6 hin gekrümmt nach außen verlaufen, um in Seitenklappen 14, 16 am Vorderbereich 4 bzw. am Rückbereich 6 überzugehen. Die Seitenklappen 14, 16 erstrecken sich mit ihren Längsrändern 18, 20 im wesentlichen wieder parallel zur Längsrichtung 10, was jedoch nicht zwingend erforderlich ist. Der Vorderbereich 4 und der Rückbereich 6 weisen desweiteren eine quer zur Längsrichtung 10 verlaufende, einen Bundendabschnitt 22 bzw. 24, der im angelegten Zustand der Windel 2 im Hüftbereich des Benutzers zu liegen kommt, begrenzenden Querrand 26, 28 auf. Der jeweilige Längsrand 12 der Windel 2 mit seinem zu den Seitenklappen 14, 16 nach außen gekrümmten Verlauf definiert im angelegten Zustand der Windel eine jeweilige Beinöffnung.

Eine die körperzugewandte Seite der Windel 2 bildende innere Oberfläche 30 ist von einer flüssigkeitsdurchlässigen Oberflächenlage 32 gebildet, die sich in Längsrichtung vom Querrand 26 bis zum Querrand 28 erstreckt. Unterhalb der flüssigkeitsdurchlässigen Oberflächenlage 32 ist ein Saugkörper 34 vorgesehen, der - wie aus Figur 1 ersichtlich - eine sanduhrförmige Umfangsform aufweist und sich von einer Querkante 36 im Vorderbereich 4 bis zu einer Querkante 38 im Rückbereich 6 erstreckt, wobei die Querkanten 36, 38 einen Abstand in der Größenordnung von cm zum jeweiligen Querrand 26 bzw. 28 aufweisen. Der Saugkörper 34 ist also in Längsrichtung 10 vollständig von der oberen flüssigkeitsdurchlässigen Oberflächenlage 32 abgedeckt. Die Oberflächenlage 32 weist zwei gerade Längsränder 40 auf, die einen solchen Abstand voneinander aufweisen, dass sie den Saugkörper 34 im Mittelbereich 8 quer zur Längsrichtung 10 hin übergreifen, wohingegen im Vorderbereich 4 sowie im Rückbereich 6, ungefähr im Bereich der Seitenklappen 14, 16, der sanduhrförmige Saugkörper 34 unter der Oberflächenlage quer zur Längsrichtung 10 hervorragt.

Eine die körperabgewandte Außenseite 42 der Windel 2 bildende Schicht 44 ist von einer zumindest in einem zentralen Längsbereich 45 flüssigkeitsdichten folienartigen Lage 46 gebildet, welche den Saugkörper 34 von außen begrenzt. Um die Erstreckung und Positionierung des Saugkörpers 34 zu fixieren, ist die obere flüssigkeitsdurchlässige Lage 32 entlang der quer verlaufenden Bundendabschnitte 22, 24 und entlang derjenigen Abschnitte der Längsränder 40, welche den Saugkörper 34 quer zur Längsrichtung 10 überlappen, mit der äußeren flüssigkeitsdichten Lage 44 verbunden, wobei hierfür ansich beliebige Verbindungen mittels Kleber oder durch Heißsiegeln oder dergleichen in Frage kommen.

Wie aus der Figur 1 desweiteren ersichtlich ist, erstrecken sich in einem Abstand zu den Längsrändern 12 und in Längsrichtung 10 erste äußere und zweite innere ansich bekannte Elastifizierungsmittel 48 bzw. 50, die äußere bzw. innere Beinabschlüsse bilden. Die äußeren und inneren Elastifizierungsmittel sind von einer hydrophoben Deckschicht 52 überfangen, die sich entlang der Längskante 12 der Windel von einem Querrand 26 zum anderen Querrand 28 erstreckt. Quer zur Längsrichtung 10 reicht die Deckschicht 52 von außen nach innen bis zu dem inneren Elastifizierungsmittel 50 und bildet dort unter Aufnahme bzw. Einschluss des inneren Elastifizierungsmittels 50 eine Falte 54, indem ein innerer längsverlaufender Randabschnitt 56 der Deckschicht 52 umgefaltet und gegen die untere Seite der Deckschicht 52 geklebt ist. Im Mittelbereich 8 der Windel 2 wird unter der Vorspannung des inneren Elastifizierungsmittels 50 auf diese Weise ein in Richtung auf die Körperoberfläche eines Benutzers aufstehendes Bündchen 58 gebildet. Dieses im Bereich beider Beinabschlüsse vorgesehene Bündchen 58 erstreckt sich bis in den Vorderbereich 4 und in den Rückbereich 6, um dort in Längsrichtung 10 auf Höhe der Seitenklappen 14, 16 durch Umlegen der inneren Seite der Falte 54 bzw. des Bündchens 58 gegen die innere Oberfläche 30 der flüssigkeitsdurchlässigen Oberflächenlage 32 festgeklebt, insbesondere dort aufgesiegelt zu werden. Das innere Elastifizierungsmittel 50 endet in Längsrichtung in einem Abstand von den Querrändern 26, 28 der Windel 2 und ist im Bereich der umgelegten und festgelegten Falte 54 ebenfalls fixiert, um so in Längsrichtung eine Spannung ausüben zu können, damit die Bündchen 58 "aufstehen". Die äußeren Elastifizierungsmittel 48 sind unter Längsspannung stehend zwischen der Deckschicht 52 und der flüssigkeitsundurchlässigen außenseitigen folienartigen Lage 46 festgeklebt, insbesondere gesiegelt. Auch sie enden in Längsrichtung 10 in einem Abstand von den Querrändern 26, 28, was jedoch nicht zwingend notwendig ist, aber einen spannungsfreien Bundendabschnitt 22 bzw. 24 schafft.

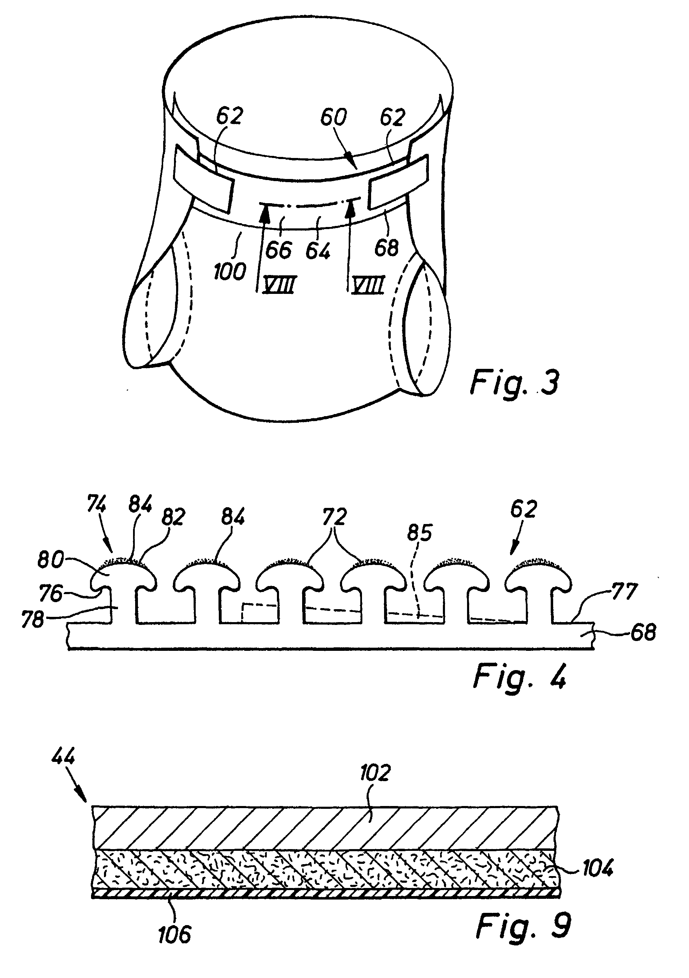

Zum Schließen der Windel im an den Benutzer angelegten Zustand ist ein insgesamt mit dem Bezugszeichen 60 bezeichnetes Befestigungssystem vorgesehen (Figur 3). Das Befestigungssystem 60 umfasst einen ersten Befestigungsabschnitt 62 an beiden Seiten des Rückbereichs 6 und einen zweiten eine Art Zielfläche für den ersten Befestigungsabschnitt 62 bildenden Befestigungsabschnitt 64, der in Form eines bandförmigen noch näher zu beschreibenden Streifens 66 im vorderen Bundendabschnitt 22 an der Außenseite des Vorderteils 4 angebracht ist.

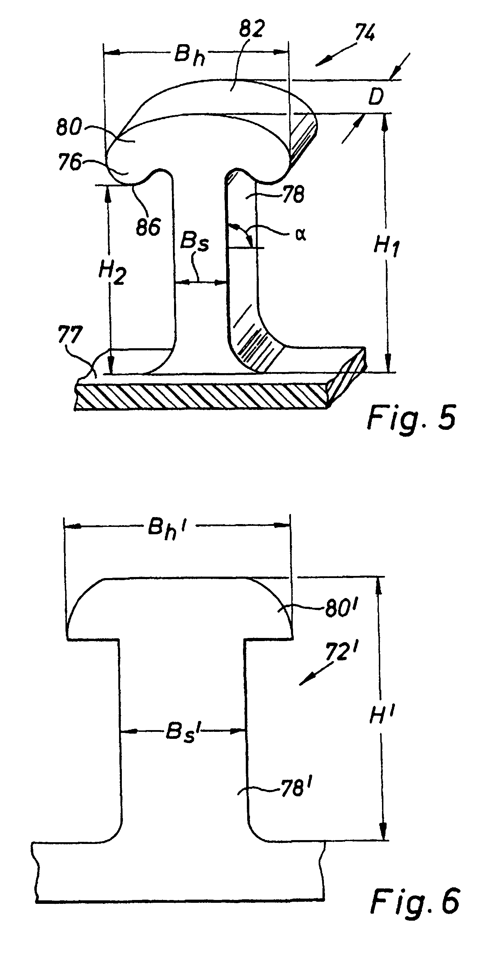

Der jeweilige Befestigungsabschnitt 62 ist von einer bandförmigen Lasche 68 gebildet. Die Lasche 68 ist ungefähr Y-förmig ausgebildet und übergreift vom Längsrand 20 der jeweiligen Seitenklappe 16 die Oberseite bzw. Unterseite der Windel 2 und ist dort festgeklebt. Die Lasche 68 weist auf ihrer im nach außen geklappten Zustand (siehe Figur 1) körperzugewandten Seite 70 mechanisch wirkende Festhaltemittel 72 auf (Figur 4, die eine schematische Schnittansicht eines die Festhaltemittel 72 aufweisenden Abschnitts der Lasche 68 zeigt). Die mechanisch wirkenden Festhaltemittel 72 sind von in der Schnittansicht T- oder pilzförmigen Vorsprüngen 74 mit hintergreifenden oder hintergreifbaren Bereichen 76 versehen. Ein einzelner derartiger Vorsprung 74 ist vergrößert in Figur 5 dargestellt. Ein solcher Vorsprung umfasst einen von einer Grundfläche 77 der Lasche 68 ausgehenden Stamm 78 und einen das freie Ende bildenden Kopf 80. Die mechanischen Festhaltemittel 72 bzw. Vorsprünge 74 weisen auf einem Teil ihrer Oberfläche 82 oder auf der gesamten Oberfläche 82 einen Klebstoffauftrag 84 in Form eines eine permanente Haftkraft aufweisenden Haftklebers auf (in Figur 5 nicht dargestellt). Anstelle eines Klebstoffauftrags können die Festhaltemittel 72 bzw. Vorsprünge 74 auch ganz aus einem permanentklebende Eigenschaften aufweisenden Material gebildet sein.

Es wäre auch denkbar, dass im Bereich zwischen den Vorsprüngen 74 auf der Grundfläche 77 ein weiterer Klebstoffauftrag 85 vorgesehen ist, wodurch die Klebekraft weiter erhöht werden kann, wenn die Lasche 68 mit großer Kraft auf einen Bereich aufgedrückt wird, zu dem der Kleber eine haftende Wirkung zeigt. Es kann sich dann - wie in der Figur 4 angedeutet - als vorteilhaft erweisen, wenn die Dicke des weiteren Klebstoffauftrags quer zur Längsrichtung 10, also in Richtung auf das freie Laschenende hin, abnimmt, so dass beim Lösen der Lasche 68 das freie Laschenende leichter gelöst werden kann.

Wie Figur 5 zu entnehmen ist, umfassen die Vorsprünge 74 eine Gesamthöhe H1 von 0,61 mm, wobei eine Distanz H2 zwischen einer Unterkante 86 des hintergreifbaren Bereichs 76 und der Grundfläche 77 der Lasche 68 0,37 mm beträgt. Die Dicke D des Stamms 78 und des Kopfs 80 beträgt 0,3 mm. Die Breite Bs des Stamms beträgt im dargestellten Fall 0,21 mm und die Breite Bh des Kopfs 80 beträgt 0,55 mm. Der Winkel α, den der Stamm 78 mit der Oberfläche 70 einschließt, beträgt 90°. Nach einem bevorzugten Ausführungsbeispiel werden 87 Vorsprünge pro cm² der Grundfläche 77 der Lasche 68 vorgesehen. Es wird darauf hingewiesen, dass die vorstehend beschriebene Form einer bevorzugten Ausführungsform entspricht, dass aber auch andere Ausbildungen der Vorsprünge möglich sind.

Figur 6 zeigt eine Ausführungsform eines pilzförmigen Festhaltemittels 72' mit kreisförmigem Querschnitt von Kopf und Stamm, dessen Kopf 80' eine Breite Bh' von 0,4 mm gegenüber einer Breite des Stamms 78' Bs' von 0,25 mm aufweist. Die Höhe des Kopfs H' gegenüber der Grundfläche beträgt 0,53 mm.

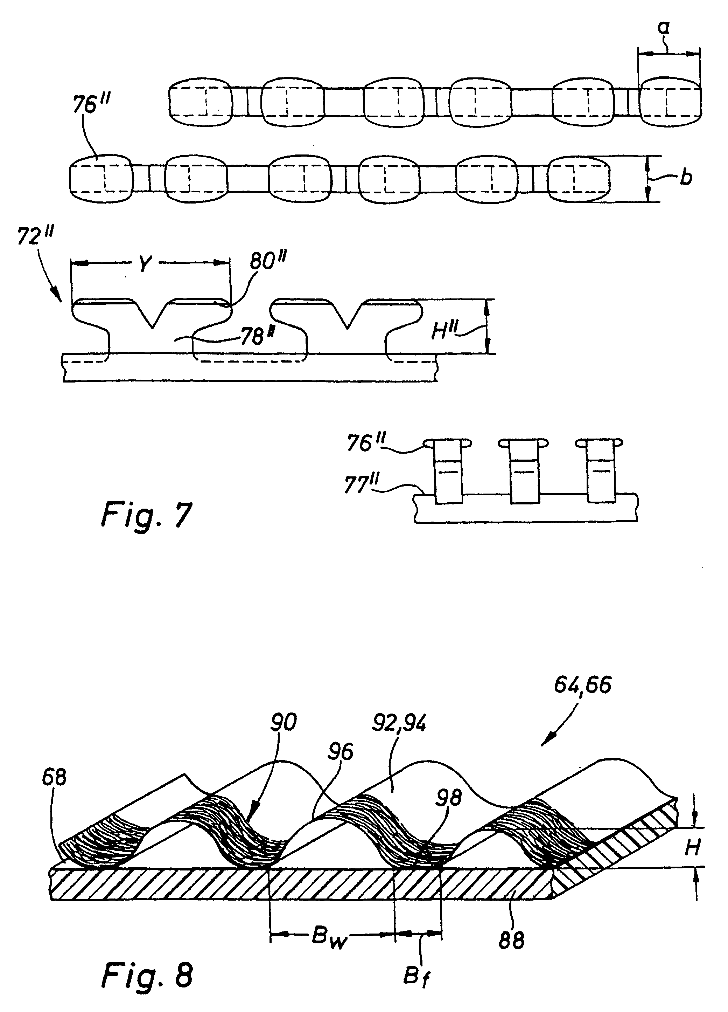

Figur 7 zeigt eine dritte Ausführungsform von Festhaltemitteln 72'', die in der Ansicht Y-förmig ausgebildet sind. Die Festhaltemittel 72'' umfassen einen Stamm 78'' mit einer Dicke von 0,15 bis 0,25 mm. Der am freien Ende der Schenkel ausgebildete Kopf 80'' bildet eine Art Tischfläche für den aufzutragenden Kleber. Diese Tischfläche weist eine bevorzugte Abmessung a, b von 0,27 mal 0,25 bis 0,35 mm auf. Der Kopf 80'' ist so ausgebildet, dass er wie aus der Figur 7 ersichtlich den Stamm 78'' in zwei entgegengesetzten Richtungen übergreift und so einen hintergreifbaren Bereich 76'' bildet. Die Höhe H'' des Kopfs über der Grundfläche 77'' beträgt etwa 0,32 mm. Die Erstreckung Y beider Schenkel der Y-förmigen Festhaltemittel 72'' beträgt 0,75 mm. Bei dieser bevorzugten Ausführungsform bildet sowohl die Abflachung als auch der Verlauf der beiden freien Schenkel der Y-Form einen hintergreifbaren Bereich. Gleichzeitig ist die tischartige Abflachung für einen Klebstoffauftrag besonders geeignet. Die bevorzugte Dichte der Y-förmigen Vorsprünge beträgt etwa 250/cm².

Der im Zusammenhang mit Figur 3 erwähnte erste Befestigungsabschnitt 62 wird zum Schließen der Windel 2 auf den zweiten Befestigungsabschnitt 64, also auf den Streifen 66 im Vorderbereich 4 aufgedrückt. Figur 8 zeigt eine Schnittansicht des Streifens 66. Dieser weist eine zweischichtige Struktur mit einer Trägerschicht 88 und einer ein Festhaltegegenmittel 90 bildenden Schlaufenschicht 92 aus einem flauschförmigen, faserigen Material 94 auf. Bei der Trägerschicht kann es sich vorzugsweise um eine Folie, ein Spinnvlies oder ein Meltblown-Vlies handeln. Die Schlaufenschicht 92 kann in vorteilhafter Weise von einem Kardenvlies oder einem Spinnvlies gebildet sein, in den die Festhaltemittel 72 des ersten Befestigungsabschnitts 62 eingreifen und verhaken können. Die Schlaufenschicht 92 weist hierfür schlaufenbildende Erhebungen 96 auf, die dadurch hergestellt sind, dass das auf die Trägerschicht 88 aufgebrachte, vorzugsweise ein Vlies bildende Nonwoven-Material 94 abschnittsweise gegen die Trägerschicht 88 fixiert ist (Bezugszeichen 98). Die Fixierung kann entlang paralleler Linien - wie in Figur 8 dargestellt - oder in Form von rauten-, dreieck- oder kreisförmigen Mustern durch einen Heißsiegelprozess erfolgen.

Für die Haftkraft ist die Ausführung der Schlaufenschicht 92 von großer Bedeutung. Bei dem in Figur 8 dargestellten Ausführungsbeispiel bilden die Erhebungen 96 endlose Wellen, wobei deren Breite Bw 2 bis 5 mm und die Breite der fixierten Fläche 98 quer zu deren Erstreckung Bf 0,2 bis 5 mm beträgt. Die Höhe H der Erhebungen 96 beträgt vorzugsweise 0,2 bis 5 mm. Es wird vorzugsweise eine Faserstärke des flauschförmigen Materials 94 von 6 bis 9 dtex und eine Faserlänge von 4 bis 5 cm gewählt. Das Flächengewicht des Materials 92 beträgt 40 bis 70 g/m². Wenn zum Schließen der Windel die Lasche 68 in korrekter Weise an dem Streifen 66 positioniert und leicht angedrückt wird, so greifen die Vorsprünge 74 in die Schlaufenschicht 92 ein und verhaken dort mechanisch, wobei eine Peel-Off-Kraft von 2 bis 4 N/25 mm, bevorzugtermaßen erreicht wird, um sicherzustellen, dass sich die Windel nicht in unbeabsichtigter Weise öffnet.

Um den Benutzer der Windel zu zwingen, die Windel in korrekter Weise zu schließen, d.h. den ersten Befestigungsabschnitt 62 in korrekter Weise an dem zweiten Befestigungsabschnitt 64 zu positionieren, damit eine optimale Passform der Windel erreicht werden kann, ist die Umgebung 100 des zweiten Befestigungsabschnitts 64 bzw. des Streifens 66 so ausgebildet, dass der erste Befestigungsabschnitt 62, wenn er darauf positioniert und angedrückt wird, eine 2. Peel-Off-Kraft, die geringer ist als die 1. Peel-Off-Kraft, aufweist. Diese 2. Peel-Off-Kraft wird im Falle einer folienartigen sehr glatten Außenseite 42 durch den Klebstoffauftrag 84 bestimmt. Es ist jedoch auch möglich, die Schicht 44, welche die körperabgewandte Außenseite 42 der Windel bildet, mit einem gewissen textilen Charakter zu versehen. Solchenfalls ist es jedoch erforderlich, dass die Außenseite 42 wenigstens in der Umgebung 100 des zweiten Befestigungsabschnitts 64 derart glatt oder geglättet ausgebildet ist und die Festhaltemittel 72 des ersten Befestigungsabschnitts 62 derart klebend sind, dass eine Peel-Off-Kraft von 2 N/25 mm, inbesondere von 1 N/25mm nicht überschritten wird. Wenn ein Benutzer der Windel den ersten Befestigungsabschnitt 62 nicht korrekt gegenüber dem zweiten Befestigungsabschnitt 64, sondern in der Umgebung 100 positioniert, so wird er durch die sehr geringe Haftkraft in der Umgebung 100 des zweiten Befestigungsabschnitts 64 sofort auf seinen Irrtum hingewiesen und wird den ersten Befestigungsabschnitt 62 abziehen und erneut, diesmal in korrekter Weise, gegenüber dem zweiten Befestigungsabschnitt 64 positionieren.

Die die Außenseite 42 bildende Schicht 44 ist in Form eines Spunbond-Meltblown-Laminats (Figur 9) ausgebildet, das eine folienähnliche Meltblown-Schicht 102, eine sich daran anschließende Spinnvliesschicht 104 und eine innen liegende Folienschicht 106 umfasst.

Wenn die benutzte Windel 2 zum Entsorgen zusammengerollt oder gefaltet wird, so kann ein Benutzer die ersten Befestigungsabschnitte 62 bzw. Laschen 68 irgendwo auf die Außenseite - es wird sich im zusammengerollten Zustand der Windel dann der Mittelbereich 6 als Zielfläche darstellen - aufdrücken, so dass diese daran zumindest noch mit einer Peel-Off-Kraft von wenigstens 0,1, vorzugsweise von 0,5 bis 1 N/25 mm, haften. Diese verhältnismäßig geringe Haftkraft hat sich für den Zweck, die Windel im zusammengerollten oder gefalteten Zustand zu fixieren, und sie in dieser Konfiguration in einen Behälter zu werfen, als hinreichend erwiesen.

## Patentansprüche

1. Hygieneartikel zum einmaligen Gebrauch, insbesondere eine Windel, ein Inkontinenzprodukt oder eine Inkontinenzeinlage, mit einem Vorderbereich (4), einem Rückbereich (6) und einem dazwischen liegenden im Schrittbereich des Benutzers des Hygieneartikels zu liegen kommenden Mittelbereich (8), die eine die körperabgewandte Außenseite (42) bildende, zumindest abschnittsweise flüssigkeitsdichte Schicht (44) und eine die körperzugewandte Innenseite (30) bildende, zumindest im Bereich eines darunter angeordneten Saugkörpers (34) flüssigkeitsdurchlässige Schicht (32) aufweisen, und mit einem Befestigungssystem (60) zum lösbaren Verbinden von Rückbereich (6) und Vorderbereich (4) des Hygieneartikels im an den Benutzer angelegten Zustand und zum Fixieren des Artikels im zusammengerollten oder gefalteten Zustand des gebrauchten Artikels beim Wegwerfen oder Entsorgen, wobei das Befestigungssystem (60) einen ersten Befestigungsabschnitt (62) mit Festhaltemitteln (72) am Rückbereich (6) und einen zweiten Befestigungsabschnitt (64) mit Festhaltegegenmitteln (90) am Vorderbereich (4) aufweist, wobei der erste Befestigungsabschnitt (62) beim Verbinden von Rückbereich (6) und Vorderbereich (4) am zweiten Befestigungsabschnitt (64) positionierbar ist, damit die Festhaltemittel (72) und Festhaltegegenmittel (90) eine mechanische Halteverbindung eingehen, und wobei die Festhaltemittel (72) des ersten Befestigungsabschnitts (62) zusätzlich klebend ausgestaltet sind, **dadurch gekennzeichnet**,
a) dass die Festhaltegegenmittel (90) des zweiten Befestigungsabschnitts (64) klebstofffrei sind und zum Schließen des Hygieneartikels im an den Körper angelegten Zustand mit den Festhaltemitteln (72) des ersten Befestigungsabschnitts (62) eine mechanische Halteverbindung mit einer 1. Peel-Off-Kraft von mehr als 1 N/25mm ausbildet.
b) dass die Festhaltemittel (72) des ersten Befestigungsabschnitts (62) derart klebend und die die Außenseite (42) bildende Schicht (44) zumindest in der Umgebung (100) des zweiten Befestigungsabschnitts (64) am Vorderbereich (4) derart glatt ausgebildet ist, dass die Festhaltemittel (72) des ersten Befestigungsabschnitts (62) dort mit einer 2. Peel-Off-Kraft haften, die geringer als die 1. Peel-Off-Kraft ist, und
c) dass die Festhaltemittel (72) des ersten Befestigungsabschnitts (62) derart klebend und die die Außenseite (42) bildende Schicht (44) zumindest im Mittelbereich (8) derart glatt ausgebildet ist, dass die Festhaltemittel (72) des ersten Befestigungsabschnitts (62) daran mit einer Peel-Off-Kraft von wenigstens 0,1 N/25mm klebend haften, um den Hygieneartikel in zusammengerollter oder -gefalteter Konfiguration zu fixieren.

2. Hygieneartikel nach Anspruch 1, **dadurch gekennzeichnet, dass** die 1. Peel-Off-Kraft 2 bis 4 N/25mm beträgt.

3. Hygieneartikel nach Anspruch 1 oder 2, **dadurch gekennzeichnet, dass** die 2. Peel-Off-Kraft 0,5 bis 2 N/25mm, insbesondere 0,5 bis 1 N/25 mm beträgt.

4. Hygieneartikel nach Anspruch 1, 2 oder 3, **dadurch gekennzeichnet, dass** die Festhaltemittel (72) von einen hintergreifbaren Abschnitt (76) aufweisenden haken-, T-oder pilzförmigen Vorsprüngen (74) mit einer Erhebung (H1) von 0,2 bis 1,5 mm senkrecht zu einer Grundfläche (77) gebildet sind.

5. Hygieneartikel nach Anspruch 4, **dadurch gekennzeichnet, dass** die Festhaltemittel (72) an ihrem freien Ende derart geformt sind, dass sie in Richtung auf die Grundfläche (77) zurückweisen.

6. Hygieneartikel nach Anspruch 4 oder 5, **dadurch gekennzeichnet, dass** die Distanz (H2) zwischen dem freien Ende der Festhaltemittel (72) und der Grundfläche (77) 0,1 bis 1,3 mm beträgt.

7. Hygieneartikel nach Anspruch 4, 5 oder 6, **dadurch gekennzeichnet, dass** die haken-, T- oder pilzförmigen Vorsprünge (74) einen von der die Außenseite bildenden Schicht ausgehenden Stamm (78) und einen an dessen freien Ende ausgebildeten Kopf (80) aufweisen, und dass die Breite (Bs) des Stamms 0,1 bis 0,4 mm und die Breite (Bh) des Kopfs 0,2 bis 0,8 mm in einer Richtung betragen.

8. Hygieneartikel nach Anspruch 7, **dadurch gekennzeichnet, dass** die gemeinsame Dicke (D) von Stamm (78) und Kopf (80) 0,1 bis 0,6 mm beträgt.

9. Hygieneartikel nach Anspruch 7 oder 8, **dadurch gekennzeichnet, dass** sich der Stamm (78) zur Grundfläche (77) in einem Winkel alpha zwischen 45 und 135 Grad erstreckt.

10. Hygieneartikel nach einem der Ansprüche 4 bis 9, **dadurch gekennzeichnet, dass** die Dichte der Festhaltemittel (72) 40 bis 400, vorzugsweise 50 bis 100 Stück je cm² beträgt.

11. Hygieneartikel nach einem der Ansprüche 7 bis 10, **dadurch gekennzeichnet, dass** die auf die Grundfläche (77) projizierte Fläche des Kopfs (80) der Festhaltemittel (72) 0,05 bis 0,5 mm² beträgt.

12. Hygieneartikel nach einem der Ansprüche 1 bis 3, **dadurch gekennzeichnet, dass** die Festhaltemittel (72'') von einen hintergreifbaren Abschnitt (76'') aufweisenden Vorsprüngen gebildet sind, die in einer Richtung in der Ebene des ersten Befestigungsabschnitts (62) gesehen eine X- oder Y-förmige Gestalt aufweisen, wobei die freien Enden abgeflacht sind.

13. Hygieneartikel nach Anspruch 12, **dadurch gekennzeichnet, dass** der abgeflachte Abschnitt einen Stamm (78'') der X-oder Y-förmigen Vorsprünge nach zwei einander entgegengesetzten Richtungen hin überragt.

14. Hygieneartikel nach Anspruch 12 oder 13, **dadurch gekennzeichnet, dass** die Abmessungen der Abflachungen in einer Richtung 0,2 bis 0,35 mm und senkrecht hierzu 0,2 - 0,4 mm betragen.

15. Hygieneartikel nach einem der Ansprüche 12 bis 14, **dadurch gekennzeichnet, dass** die Erstreckung des X- oder Y-förmigen Vorsprungs in Draufsicht auf den ersten Befestigungsabschnitt (62) 0,6 bis 0,9 mm beträgt.

16. Hygienartikel nach einem der Ansprüche 12 bis 15, **dadurch gekennzeichnet, dass** die Dicke des Stamms (78'') 0,15 bis 0,25 mm beträgt.

17. Hygieneartikel nach einem der Ansprüche 12 bis 16, **dadurch gekennzeichnet, dass** die Dicke des hintergreifbaren Abschnitts des Kopfs (80'') 0,1 - 0,3 mm beträgt.

18. Hygieneartikel nach einem der Ansprüche 12 bis 17, **dadurch gekennzeichnet, dass** die Distanz (H'') zwischen dem freien Ende der Festhaltemittel (72'') und der Grundfläche 0,25 bis 0,4 mm beträgt.

19. Hygieneartikel nach einem der vorstehenden Ansprüche, **dadurch gekennzeichnet, dass** der zweite Befestigungsabschnitt (64) ein die Festhaltegegenmittel (90) bildendes flauschförmiges faseriges Material (94) umfasst.

20. Hygieneartikel nach einem der vorstehenden Ansprüche, **dadurch gekennzeichnet, dass** der zweite Befestigungsabschnitt (64) eine zweischichtige Struktur aufweist mit einer Trägerschicht (88) und einer die Festhaltegegenmittel (90) bildenden Schlaufenschicht (92), in welche die Festhaltemittel (72) des ersten Befestigungsabschnitts (62) eingreifen und verhaken.

21. Hygieneartikel nach Anspruch 19 oder 20, **dadurch gekennzeichnet, dass** das faserige Material (94) eine Faserstärke von 3 bis 15 dtex, vorzugsweise 6 bis 9 dtex, aufweist.

22. Hygienartikel nach Anspruch 19, 20 oder 21, **dadurch gekennzeichnet, dass** die Faserlänge 2 bis 10 cm, vorzugsweise 4 bis 5 cm beträgt.

23. Hygieneartikel nach einem der Ansprüche 19 bis 22, **dadurch gekennzeichnet, dass** das Flächengewicht des flauschförmigen faserigen Materials (94) 15 bis 120 g/cm², vorzugsweise 40 bis 70 g/cm², beträgt.

24. Hygieneartikel nach einem der Ansprüche 19 bis 23, **dadurch gekennzeichnet, dass** das flauschförmige faserige Material (94) in einem regelmäßigen Muster angeordnete schlaufenbildende Erhebungen (96) aufweist.

25. Hygieneartikel nach Anspruch 24, **dadurch gekennzeichnet, dass** die Dicke der schlaufenbildenden Erhebungen (96) 0,2 bis 5mm beträgt.

26. Hygieneartikel nach Anspruch 24 oder 25, **dadurch gekennzeichnet, dass** die schlaufenbildenden Erhebungen (96) durch Heißsiegeln Kleben oder Schweißen des flauschförmigen faserigen Materials (94) gebildet sind und die Breite (Bf) der Heißsiegelspur bzw. der Kleber- oder Schweißspur 0,1-10 mm, vorzugsweise 0,2-5 mm beträgt.

27. Hygieneartikel nach Anspruch 24, 25 oder 26, **dadurch gekennzeichnet, dass** die Breite (Bw) der schlaufenbildenden Erhebungen (96) 1 bis 10 mm, vorzugsweise 2 bis 5 mm beträgt.

28. Hygieneartikel nach einem oder mehreren der vorstehenden Ansprüche, **dadurch gekennzeichnet, dass** die die körperabgewandte Außenseite (42) bildende Schicht (44) eine Folie ist.

29. Hygieneartikel nach einem oder mehreren der vorstehenden Ansprüche, **dadurch gekennzeichnet, dass** die die körperabgewandte Außenseite (42) bildende Schicht (44) ein zumindest zweischichtiges Vlies-Folien-Laminat umfasst.

30. Hygieneartikel nach Anspruch 29, **dadurch gekennzeichnet, dass** die die körperabgewandte Außenseite (42) bildende Schicht (44) ein dreischichtiges Laminat aus einer die Außenseite (42) bildenden Meltblown-Schicht (102) und einer sich daran anschließenden Spinnvlies-Schicht (104) und einer innen liegenden Folienschicht (106) umfasst.

31. Hygieneartikel nach Anspruch 30, **dadurch gekennzeichnet, dass** das Flächengewicht der Spinnvlies-Schicht (104) 10 bis 25 g/m² beträgt.

32. Hygieneartikel nach Anspruch 30 oder 31, **dadurch gekennzeichnet, dass** das Flächengewicht der Meltblown-Schicht (102) 3 bis 15 g/m² beträgt.

33. Hygieneartikel nach Anspruch 30, 31 oder 32, **dadurch gekennzeichnet, dass** die Dicke der Folienschicht (106) 5 bis 25 µm beträgt.

## Claims

1. Disposable sanitary article, in particular a nappy, an incontinence product or an incontinence insert, with a front region (4), a back region (6) and a middle region (8) located therebetween and coming to rest in the crotch region of the user of the sanitary article, comprising a layer (44) which is liquid-tight at least in sections and forms the outer side (42) remote from the body, and a layer (32) which is liquid-permeable at least in the region of an absorbent member (34) arranged therebelow and forming the inner side (30) facing the body, and with a fastening system (60) for detachable connection of back region (6) and front region (4) of the sanitary article when placed on the user and for fixing the article in the rolled up or folded state of the used article when thrown away or disposed, the fastening system (60) comprising a first fastening portion (62) with fastening means (72) on the back region (6) and a second fastening portion (64) with counter-fastening means (90) on the front region (4), wherein the first fastening portion (62) can be positioned on the second fastening portion (64) when connecting back region (6) and front region (4), so the fastening means (72) and counter-fastening means (90) enter a mechanical holding connection, the fixing means (72) of the first fastening portion (62) additionally being designed so as to be adhesive, **characterised in that**
a) the counter-fastening means (90) of the second fastening portion (64) are free of adhesive and to close the sanitary article when placed on the body form a mechanical holding connection with a first peel-off force of more than 1 N/25 mm with the fixing means (72) of the first fastening portion (62),
b) the fixing means (72) of the first fastening portion (62) is designed so as to be adhesive and the layer (44) forming the outer side (42) is designed so as to be smooth, at least in the environment (100) of the second fastening portion (64) on the front region (4), in such a way that the fixing means (72) of the first fastening portion (62) adhere there with a second peel-off force which is smaller than the first peel-off force, and
c) the fixing means (72) of the first fastening portion (62) is designed so as to be adhesive and the layer (44) forming the outer side (42) is designed so as to be smooth, at least in the middle region (8), in such a way that the fixing means (72) of the first fastening portion (62) adhere thereto in an adhesive manner with a peel-off force of at least 0.1 N/25 mm to fix the sanitary article in the rolled up or folded up configuration.

2. Sanitary article according to claim 1, **characterised in that** the first peel-off force is 2 to 4 N/25 mm.

3. Sanitary article according to claim 1 or 2, **characterised in that** the second peel-off force is 0.5 to 2 N/25 mm, in particular 0.5 to 1 N/25 mm.

4. Sanitary article according to claim 1, 2 or 3, **characterised in that** the fixing means (72) are formed by hook-, T- or mushroom-shaped projections (74) comprising an engageable portion (76) with an elevation (H1) of 0.2 to 1.5 mm perpendicular to a base (77).

5. Sanitary article according to claim 4, **characterised in that** the fixing means (72) are designed at their free end such that they point back in the direction of the base (77).

6. Sanitary article according to claim 4 or 5, **characterised in that** the distance (H2) between the free end of the fixing means (72) and the base (77) is 0.1 to 1.3 mm.

7. Sanitary article according to claim 4, 5 or 6, **characterised in that** the hook-, T- or mushroom-shaped projections (74) have a stem (78) issuing from the layer forming the outer side and a head (80) formed at the free end of the stem, and **in that** the width (Bs) of the stem is 0.1 to 0.4 mm and the width (Bh) of the head is 0.2 to 0.8 mm in one direction.

8. Sanitary article according to claim 7, **characterised in that** the common thickness (D) of stem (78) and head (80) is 0.1 to 0.6 mm.

9. Sanitary article according to claim 7 or 8, **characterised in that** the stem (78) extends toward the base (77) at an angle alpha between 45 and 135 degrees.

10. Sanitary article according to any of claims 4 to 9, **characterised in that** the density of the fixing means (72) is 40 to 400, preferably 50 to 100 pieces per cm².

11. Sanitary article according to any of claims 7 to 10, **characterised in that** the area of the head (80) of the fixing means (72) projected onto the base (77) is 0.05 to 0.5 mm².

12. Sanitary article according to any of claims 1 to 3, **characterised in that** the fixing means (72'') are formed by projections comprising an engageable portion (76'') and having an X- or Y-shaped form, as viewed in a direction in the plane of the first fastening portion (62), the free ends being flattened.

13. Sanitary article according to claim 12, **characterised in that** the flattened portion projects beyond a stem (78'') of the X- of Y-shaped projections in two opposing directions.

14. Sanitary article according to claim 12 or 13, **characterised in that** the dimensions of the flattenings are 0.2 to 0.35 mm in one direction and 0.2 to 0.4 mm perpendicular thereto.

15. Sanitary article according to any of claims 12 to 14, **characterised in that** the extension of the X- or Y-shaped projection is 0.6 to 0.9 mm in the plan view of the first fastening portion (62).

16. Sanitary article according to any of claims 12 to 15, **characterised in that** the thickness of the stem (78'') is 0.15 to 0.25 mm.

17. Sanitary article according to any of claims 12 to 16, **characterised in that** the thickness of the engageable portion of the head (80'') is 0.1 to 0.3 mm.

18. Sanitary article according to any of claims 12 to 17, **characterised in that** the distance (H'') between the free end of the fixing means (72'') and the base is 0.25 to 0.4 mm.

19. Sanitary article according to any of the preceding claims, **characterised in that** the second fastening portion (64) comprises a fleecy fibrous material (94) forming the counter-fastening means (90).

20. Sanitary article according to any of the preceding claims, **characterised in that** the second fastening portion (64) comprises a two-layered structure with a carrier layer (88) and a looped layer (92) forming the counter-fastening means (90) in which the fixing means (72) of the first fastening portion (62) engage and interlock.

21. Sanitary article according to claim 19 or 20, **characterised in that** the fibrous material (94) has a fibre thickness of 3 to 15 dtex, preferably 6 to 9 dtex.

22. Sanitary article according to claim 19, 20 or 21, **characterised in that** the fibre length is 2 to 10 cm, preferably 4 to 5 cm.

23. Sanitary article according to any of claims 19 to 22, **characterised in that** the mass per unit area of the fleecy fibrous material (94) is 15 to 120 g/cm², preferably 40 to 70 g/cm².

24. Sanitary article according to any of claims 19 to 23, **characterised in that** the fleecy fibrous material (94) has loop-forming elevations (96) arranged in a regular pattern.

25. Sanitary article according to claim 24, **characterised in that** the thickness of the loop-forming elevations (96) is 0.2 to 5 mm.

26. Sanitary article according to claim 24 or 25, **characterised in that** the loop-forming elevations (96) are formed by heat sealing, gluing or welding the fleecy fibrous material (94) and the width (Bf) of the heat seal track or the glue or weld track is 0.1 to 10 mm, preferably 0.2 to 5 mm.

27. Sanitary article according to claim 24, 25 or 26, **characterised in that** the width (Bw) of the loop-forming elevations (96) is 1 to 10 mm, preferably 2 to 5 mm.

28. Sanitary article according to any one or more of the preceding claims, **characterised in that** the layer (44) forming the outer side (42) remote from the body is a film.

29. Sanitary article according to any one or more of the preceding claims, **characterised in that** the layer (44) forming the outer side (42) remote from the body comprises an at least two-layered nonwoven-film laminate.

30. Sanitary article according to claim 29, **characterised in that** the layer (44) forming the outer side (42) remote from the body comprises a three-layered laminate made of a meltblown layer (102) forming the outer side (42) and an adjoining spun bonded layer (104) and an inner film layer (106).

31. Sanitary article according to claim 30, **characterised in that** the mass per unit area of the spun bonded layer (104) is 10 to 25 g/m².

32. Sanitary article according to claim 30 or 31, **characterised in that** the mass per unit area of the meltblown layer (102) is 3 to 15 g/m².

33. Sanitary article according to claim 30, 31 or 32, **characterised in that** the thickness of the film layer (106) is 5 to 25µm.

## Revendications

1. Article d'hygiène à usage unique, en particulier une couche, un produit pour l'incontinence ou une garniture pour l'incontinence, comportant une zone avant (4), une zone arrière (6) et une zone médiane (8) qui vient se placer entre les deux autres zones, dans la zone de marche de l'utilisateur de l'article d'hygiène, lesdites zones comportant une couche (44), qui est, au moins par sections, étanche aux liquides et qui forme la face extérieure (42) dirigée vers le corps, et une couche (32) qui est perméable aux liquides au moins dans la zone d'un corps absorbant (34) disposé en dessous, ledit article d'hygiène comportant un système de fixation (60) pour réaliser la liaison amovible de la zone arrière (6) et de la zone avant (4) de l'article d'hygiène lorsqu'il est placé sur l'utilisateur et pour fixer l'article lorsqu'il est à l'état enroulé ou replié pour être jeté ou évacué, article dans lequel le système de fixation (60) comporte une première section de fixation (62) comprenant des moyens de fixation (72) disposés dans la zone arrière (6) et une deuxième section de fixation (64) comprenant des moyens de contre-fixation (90) disposés dans la zone avant (4), dans lequel la première section de fixation (62) peut être positionnée sur la deuxième section de fixation (64) lors de la mise en liaison de la zone arrière (6) et de la zone avant (4) pour que les moyens de fixation (72) et les moyens de contre-fixation (90) réalisent une liaison mécanique de maintien, et dans lequel les moyens de fixation (72) de la première section de fixation (62) sont, de plus, réalisés sous forme adhésive, **caractérisé en ce que** :
a) les moyens de contre-fixation (90) de la deuxième section de fixation (64) sont exempts de matière adhésive et, pour fermer l'article d'hygiène lorsqu'il est placé sur le corps au moyen des moyens de fixation (72) de la première section de fixation (62), on forme une liaison mécanique de maintien qui a une première force d'arrachage (Peel Off) supérieure à 1 N/25 mm
b) les moyens de fixation (72) de la première section de fixation (62) sont adhésifs de manière telle, et la couche (44) formant la face extérieure (42) est conformée lisse, au moins dans la périphérie (100) de la deuxième section de fixation (64), dans la zone avant (4), de manière telle, que les moyens de fixation (72) de la première section de fixation (62) s'y accrochent avec une deuxième force d'arrachage plus faible que la première force d'arrachage ; et
c) les moyens de fixation (72) de la première section de fixation (62) sont adhésifs de manière telle, et la couche (44) formant la face extérieure (42) est conformée lisse, au moins dans la zone médiane (8), dans la zone avant (4), de manière telle, que les moyens de fixation (72) de la première section de fixation (62) s'y accrochent par collage avec une force d'arrachage égale à au moins 0,1 N/25 mm pour fixer l'article d'hygiène dans sa configuration enroulée ou repliée.

2. Article d'hygiène selon la revendication 1, **caractérisé en ce que** la première force d'arrachage est comprise entre 2 à 4 N/25 mm.

3. Article d'hygiène selon la revendication 1 ou 2, **caractérisé en ce que** la deuxième force d'arrachage est comprise entre 0,5 à 1 N/25 mm, de préférence entre 0,5 et 1 N/25 mm.

4. Article d'hygiène selon la revendication 1, 2 ou 3, **caractérisé en ce que** les moyens de fixation (72) sont constitués par des parties en saillie (74) qui sont en forme de crochet, de « T » ou de champignon, qui comportent une section (76) pouvant être saisie par l'arrière et qui ont une élévation (H1) comprise entre 0,1 et 1,5 mm perpendiculairement par rapport à une surface de base (77).

5. Article d'hygiène selon la revendication 4, **caractérisé en ce que** les moyens de fixation (72) sont conformés, à leur extrémité libre, de telle manière qu'ils se retournent vers la surface de base (77).

6. Article d'hygiène selon la revendication 4 ou 5, **caractérisé en ce que** la distance (H2) entre l'extrémité libre des moyens de fixation (72) et la surface de base (77) est comprise entre 0,1 et 1,3 mm.

7. Article d'hygiène selon la revendication 4, 5 ou 6, **caractérisé en ce que** les parties en saillie (74), qui sont en forme de crochet, de « T » ou de champignon, comportent une tige (78) qui ressort de la couche formant la face extérieure et une tête (80) formée sur l'extrémité libre de ce dernier, et **en ce que** la largeur (Bs) de la tige est comprise entre 0,1 et 0, 4 mm et la largeur (Bh) de la tête est comprise entre 0,2 et 0,8 mm.

8. Article d'hygiène selon la revendication 7, **caractérisé en ce que** l'épaisseur commune (D) de la tige (78) et de la tête (80) est comprise entre 0,1 et 0,6 mm.

9. Article d'hygiène selon la revendication 7 ou 8, **caractérisé en ce que** la tige (78) s'étend, par rapport à la surface de base (77), selon un angle alpha compris entre 45 et 135 degrés.

10. Article d'hygiène selon l'une quelconque des revendications 4 à 9, **caractérisé en ce que** la densité des moyens de fixation (72) est comprise entre 40 à 400, de préférence entre 50 à 100 pièces par cm².

11. Article d'hygiène selon l'une quelconque des revendications 7 à 10, **caractérisé en ce que** la surface, en projection sur la surface de base (77), de la tête (80) des moyens de fixation (72) est comprise entre 0,05 et 0,5 mm².

12. Article d'hygiène selon l'une quelconque des revendications 1 à 3, **caractérisé en ce que** les moyens de fixation (72") sont constitués par des parties saillantes qui comportent une section (76") pouvant être saisie par l'arrière et qui, vues selon une direction se trouvant dans le plan de la première section de fixation (62), présentent une structure en forme de « X » ou de « Y », leurs extrémités libres étant aplaties.

13. Article d'hygiène selon la revendication 12, **caractérisé en ce que** la section aplatie fait saillie d'une tige (78") des parties saillantes en forme de « X » ou de « Y » selon deux directions opposées l'une à l'autre.

14. Article d'hygiène selon la revendication 12 ou 13, **caractérisé en ce que** les dimensions des aplatissements sont comprises entre 0,2 à 0,35 mm, selon une direction, et entre 0,2 à 0,4 mm, selon une direction perpendiculaire à cette dernière.

15. Article d'hygiène selon l'une quelconque des revendications 12 à 14, **caractérisé en ce que** l'extension de la partie saillante en forme de « X » ou de « Y » en vue de dessus sur la première section de fixation (62) est comprise entre 0,6 à 0,9 mm.

16. Article d'hygiène selon l'une quelconque des revendications 12 à 15, **caractérisé en ce que** l'épaisseur de la tige (78") est comprise entre 0,15 à 0,25 mm.

17. Article d'hygiène selon l'une quelconque des revendications 12 à 16, **caractérisé en ce que** l'épaisseur de la section de la tête (80") qui peut être saisie par l'arrière est comprise entre 0,1 à 0,3 mm.

18. Article d'hygiène selon l'une quelconque des revendications 12 à 17, **caractérisé en ce que** la distance (H") entre l'extrémité libre des moyens de fixation (72") et l'extrémité libre de la surface de base (77) est comprise entre 0,25 à 0,4 mm.

19. Article d'hygiène selon l'une quelconque des revendications précédentes, **caractérisé en ce que** la deuxième section de fixation (64) comprend une matière (94) fibreuse en forme de tissu à longs poils qui constitue les moyens de contre-fixation (90).

20. Article d'hygiène selon l'une quelconque des revendications précédentes, **caractérisé en ce que** la deuxième section de fixation (64) comporte une structure à deux couches comportant une couche porteuse (88) et une couche (92) en forme de boucle qui constitue les moyens de contre-fixation (90) et dans laquelle les moyens de fixation (72) s'accrochent et se verrouillent.

21. Article d'hygiène selon la revendication 19 ou 20, **caractérisé en ce que** la matière fibreuse (94) a une densité de fibres comprise entre 3 à 15 dtex, de préférence entre 6 à 9 dtex.

22. Article d'hygiène selon la revendication 19, 20 ou 21, **caractérisé en ce que** la longueur des fibres est comprise entre 2 et 10 cm, de préférence entre 4 et 5 cm.

23. Article d'hygiène selon l'une quelconque des revendications 19 à 22, **caractérisé en ce que** le poids surfacique de la matière (94) fibreuse en forme de tissu à longs poils est comprise entre 15 à 120 g/cm², de préférence entre 40 à 70 g/cm².

24. Article d'hygiène selon l'une quelconque des revendications 19 à 23, **caractérisé en ce que** la matière (94) fibreuse en forme de tissu à longs poils comporte des bosses (96) formant des boucles et disposées selon un modèle régulier.

25. Article d'hygiène selon la revendication 24, **caractérisé en ce que** l'épaisseur des bosses (96) formant des boucles est comprise entre 0,2 et 5 mm.

26. Article d'hygiène selon la revendication 24 ou 25, **caractérisé en ce que** les bosses (96) formant des boucles sont formées par thermoscellement, collage ou soudage de la matière (94) fibreuse en forme de tissu à longs poils et **en ce que** la largeur (Bf) du cordon de thermoscellement, de collage ou de soudage est comprise entre 0,1 à 10 mm, de préférence entre 0,2 et 5 mm.

27. Article d'hygiène selon la revendication 24, 25 ou 26, **caractérisé en ce que** la largeur (Bw) des bosses (96) formant des boucles est comprise entre 1 et 10 mm, de préférence entre 2 et 5 mm.

28. Article d'hygiène selon l'une ou plusieurs des revendications précédentes, **caractérisé en ce que** la couche (44) formant la face extérieure (42) dirigée vers le corps est une feuille.

29. Article d'hygiène selon l'une ou plusieurs des revendications précédentes, **caractérisé en ce que** la couche (44) formant la face extérieure (42) dirigée vers le corps comprend un stratifié de feuilles en matière non tissée.

30. Article d'hygiène selon la revendication 29, **caractérisé en ce que** la couche (44) formant la face extérieure (42) dirigée vers le corps comprend un stratifié à trois couches, constitué par une couche (102) de matière fondue soufflée (meltblown) constituant la face extérieure (42), par une couche (104) de monofils continus qui s'y raccorde et par une couche de feuille (106) disposée à l'intérieur.

31. Article d'hygiène selon la revendication 30, **caractérisé en ce que** la densité surfacique de la couche (104) de monofils continus est comprise entre 10 et 25 g/m².

32. Article d'hygiène selon la revendication 30 ou 31, **caractérisé en ce que** la densité surfacique de la couche (102) de matière fondue soufflée (meltblown) est comprise entre 3 et 15 g/m².

33. Article d'hygiène selon la revendication 30, 31 ou 32, **caractérisé en ce que** l'épaisseur de la couche de feuille (106) est comprise entre 5 et 25 µm.
